# EUROPEAN PATENT SPECIFICATION

(11) **EP 1 647 592 B1**
(45) Date of publication and mention of the grant of the patent: **11.03.2009**
(21) Application number: 04746993.7
(22) Date of filing: 29.06.2004
(51) Int. Cl.: C12N 15/00, C12P 19/34, C12Q 1/68

(54) **METHOD OF MODIFYING NUCLEOTIDE CHAIN**
VERFAHREN ZUR MODIFIZIERUNG EINER NUKLEOTIDKETTE
PROCEDE DE MODIFICATION D'UNE CHAINE NUCLEOTIDE

(30) Priority: 30.06.2003 JP 2003186151; 25.06.2004 JP 2004187133
(43) Date of publication of application: 19.04.2006
(73) Proprietor: Panasonic Corporation, Kadoma-shi Osaka 571-8501 (JP)
(72) Inventor: JOKO, Shigeki, Ehime 792-0805 (JP)
(74) Representative: Grünecker, Kinkeldey, Stockmair & Schwanhäusser Anwaltssozietät
(86) International application number: PCT/JP2004/009524
(87) International publication number: WO 2005/014808

(56) References cited:
- EP-A- 1 035 214
- WO-A1-00/58329
- WO-A1-89/06698
- WO-A1-99/54501
- WO-A2-02/36821
- WO-A2-03/012100
- JP-A- 2003 246 794
- PATENT ABSTRACTS OF JAPAN vol. 2003, no. 12, 5 December 2003 (2003-12-05) & JP 2003 246794 A (MATSUSHITA KOTOBUKI ELECTRONICS INDUSTRIES LTD), 2 September 2003 (2003-09-02)
- FORGET D ET AL: "3'-Oligonucleotides conjugation via chemoselective oxime bond formation" TETRAHEDRON LETTERS, ELSEVIER, AMSTERDAM, NL, vol. 42, no. 52, 24 December 2001 (2001-12-24), pages 9171-9174, XP004328105 ISSN: 0040-4039
- TAKAHASHI T.: 'In situ hybridization no tameno oligonucleotide probe dai 3 kai oligonucleotide probe no hihoshasei hyoshiki' BIO. IND. vol. 14, no. 4, 1997, pages 26 - 45, XP002996265

### SEQUENCE LISTING

<110> MATSUSHITA ELECTRIC INDUSTRIAL CO., LTD.
<120> Method for modifying nucleotides
<130> pct4020
<150> JP 2004-187133
   <151> 2004-06-25
<150> JP 2003-186151
   <151> 2003-06-30
<160> 3
<170> PatentIn version 3. 1
<210> 1
   <211> 30
   <212> DNA
   <213> Artificial
<220>
   <223> Sequence coding for the region 8-37 of 16S rRNA gene of Escherich ia coli
<400> 1
   agagtttgat catggctcag attgaacgct 30
<210> 2
   <211> 20
   <212> DNA
   <213> Artificial
<220>
   <223> Human adult myosin heavy chain 1 coding oligodeoxynucleotide for the region 5831 to 5850
<400> 2
   tgctgaaagg tgaccaaaga 20
<210> 3
   <211>. 29
   <212> DNA
   <213> Artificial
<220>
   <223> Complementary sequence for the region 5850 to 5831 of human adult myosin heavy chain 1 in addition to 9 nucleotides that recognise Bam HI ristriction endonuclease in the 5'-terminal
<400> 3
   atcggatcct ctttggtcac ctttcagca 29

## Description

### Technical Field

The present invention relates to a method for modifying a nucleotide chain, and particularly to a method for labeling, conjugating, and immobilizing a nucleotide chain by directly modifying the 3'-terminus of the nucleotide chain with a modifier.

### Background Art

In the field of gene analysis, radioisotopes have previously been adopted as modifiers for labeling or conjugating a nucleotide chain such as DNA, RNA, oligonucleotide, or nucleic acid. However, radioisotopes have been problematic in terms of a limited duration of use due to its half-life, a limited place of use, problems regarding radioactive exposure, problems regarding disposal, and the like. Thus, the use thereof has tended to decrease. In recent years, a method for labeling or conjugating a nucleotide chain by modifying it using a fluorescent substance such as fluorescein, biotin, or the like, as modifiers that substitute for radioisotopes, has widely been used. Methods for modifying a nucleotide chain are broadly classified into 3 types: 5'-terminal modification method, 3'-terminal modification method, and internal modification method.

As such 5'-terminal modification method, a method for modifying a nucleotide chain with biotin via a phosphate group existing at the 5'-terminus has been proposed (refer to Non-Patent Document 1 indicated below, for example). Regarding biotin, methods for achieving highly sensitive gene analysis using biotin have been reported (refer to Non-Patent Documents 2 and 3, for example). Utilizing a high affinity of biotin with avidin, these methods achieve highly sensitive gene analysis by modifying a nucleotide chain with enzymes such as alkaline phosphatase or horseradish peroxidase and using chemoluminescence generated from these enzymes. In addition to the aforementioned methods, many other methods for modifying a nucleotide chain via a phosphate group existing at the 5'-terminus thereof have been proposed (refer to Patent Document 1 and Non-Patent Documents 4 to 13, for example).

However, in such methods for modifying a nucleotide chain via a phosphate group existing at the 5'-terminus, the number of modifiers applied to a nucleotide chain is basically one. Since alkaline phosphatase dissociates such a phosphate group existing at the 5'-terminus, in order to realize high sensitivity, it is necessary to basically avoid the application of alkaline phosphatase in combination with such a phosphate group. In addition, since alkaline phosphatase also exists in bacteria suspended in the air, when it contaminates a nucleotide chain during gene analysis, a modifier binding to a phosphate group existing at the 5'-terminus is likely to be dissociated, thereby resulting in an unstable modification state. Thus, alkaline phosphatase may also be a factor of background noise. Moreover, phosphoramidite method is a 5'-terminal modification method that is frequently used. In thismethod, the reaction does not completely progress. Hence, it is necessary to separate and eliminate unreacted products by liquid chromatography or the like, and thus this method involves complicated operations.

As internal modification method, random primer method and nick-translation method have been developed (refer to Patent Document 4 and Non-Patent Documents 20 to 22, for example). These are methods for labeling or conjugating a nucleotide chain by incorporating into the nucleotide chain deoxynucleotide triphosphate that has previously been modified with a modifier for labeling or conjugating the target, during the reaction of replicating the nucleotide chain.

When this internal modification method is used in combination with what is called the PCR method, polymerase chain reaction that is a gene amplification reaction (refer to Patent Documents 5 to 8 and Non-Patent Documents 23 to 25, for example), it enables amplification of a nucleotide chain and labeling or conjugating thereof, simultaneously (refer to Non-Patent Document 26, for example). The internal modification method thereby achieves highly sensitive gene analysis and realizes a high throughput of gene analysis including gene microarray as a representative example (refer to Patent Document 9 and Non-Patent Documents 27 to 29, for example).

In the aforementioned random primer method, nick-translation method, and PCR method, from the viewpoint of nucleotide uptake efficiency, synthesis of a modified nucleotide chain, etc., the formation of a nucleotide chain, into which uracil or uracil that had previously been labeled or conjugated with a fluorescent substance or the like has been incorporated instead of thymidine, has frequently been applied. Moreover, there are some other methods of making use of the advantage of using uracil instead of thymidine, which comprise incorporating uracil into a nucleotide chain caused by external contamination mixed in a reaction mixture and then allowing uracil DNA glycosylase to act thereon, so as to prevent amplification of the nucleotide chain caused by contamination during PCR (refer to Patent Documents 11 to 13 and Non-Patent Documents 31 to 33).

However, in the case of incorporating modifiers into a nucleotide chain in the PCR method, the number of modifiers incorporated is random such as a number between 10 and 20, or approximately 30 (refer to Non-Patent Document 30, for example). Thus, complete quantification has not yet been achieved. Moreover, since the labeling or conjugating treatment of deoxynucleotide triphosphate is complicated, a certain labeled or conjugated product cannot easily be obtained by anybody. As a result, the type of a modifier is limited. Furthermore, since a modifier is incorporated into nucleotides, steric hindrance inevitably occurs due to such a modifier during hybridization in gene analysis, that is, during the formation of a double-stranded nucleotide. Still further, the internal modification method is disadvantageous also in that the use thereof is limited to labeling of a nucleotide chain with a fluorescent substance or the like at an initial stage.

As 3'-terminal modification method, tailing method has been developed. This method comprises: synthesizing deoxynucleotide triphosphate or dideoxynucleotide triphosphate that has previously been subjected to a labeling or conjugating treatment (refer to Non-Patent Document 14 and Patent Document 2, for example); and adding the thus labeled or conjugated nucleotide to the 3'-terminal side of a nucleotide chain using terminal deoxynucleotidyltransferase (refer to Patent Document 3 andNon-Patent Documents 15 to 18, for example). This 3'-terminal modification method enables modification of a nucleotide chain depending on the situation. This 3'-terminal modification method has higher stability in terms of retention of a modifier than the 5'-terminal modification method, and it is preferable as a modification site. Thus, this method has widely been used.

However, since a treatment for labeling or conjugating deoxynucleotide triphosphate or dideoxynucleotide triphosphate is complicated, a certain labeled or conjugated product cannot easily be obtained by anybody. As a result, the type of a modifier is limited. In addition, since an unnecessary nucleotide sequence is added to the 3'-terminal side of a nucleotide chain at a tailing stage, in particular when deoxynucleotide triphosphate is used, the number of nucleotides added becomes random depending on reaction conditions (refer to Non-Patent Document 19, for example). Accordingly, this method realizes high sensitivity in gene analysis, but it is still problematic in terms of quantification. Such an unnecessary nucleotide sequence added to the 3'-terminal side may also become a factor of mismatch during hybridization in gene analysis.

In terms of addition of unnecessary nucleotides, Patent Document 10 proposes decomposition of a glycosidic bond of the added uracil with uracil DNA glycosylase. However, in the aforementioned random primer method, nick-translation method, and PCR method, the formation of a nucleotide chain that incorporates uracil instead of thymidine has often been applied. In addition, during PCR, uracil used for preventing the amplification of a nucleotide chain caused by contamination is incorporated also in a nucleotide chain to be amplified. Accordingly, if the method described in Patent Document 10 is applied to a nucleotide chain containing uracil, uracil DNA glycosylase acts not only on uracil added to the 3'-terminal side but also on uracil contained in a nucleotide chain as a target for modification. As a result, a nucleotide chain itself is decomposed.

As another 3'-terminal modification method, there is a method of allowing a modifier for labeling or conjugating the target to directly bind to the nucleotide at the 3'-end at an initial stage of synthesis of a chemically synthesized nucleotide chain. This method realizes high stability in terms of attachment of such a modifier to a nucleotide chain. However, the number of nucleotides obtained by chemical synthesis is approximately 100 because of synthetic ability such as synthetic by-products or yield. Thus, the method of directly binding a modifier to the 3'-end used herein cannot be applied to modification of the 3'-end of 100 or more nucleotides, which have been extracted from a living body or amplified by PCR or the like. That is to say, the length of a nucleotide chain as a target for modification is limited.

Moreover, it is difficult for these 3 types of modification methods to selectively modify only a single nucleotide chain, when nucleotide chains are present in the form of a double strand. That is, when a nucleotide chain is labeled or conjugated by the conventional methods, both nucleotide chains, which are generally present in the form of a double strand, are simultaneously modified. It is difficult to selectively modify only a single nucleotide chain or to separate and eliminate the other nucleotide chain. Thus, when gene information contained in only a single nucleotide chain out of two chains is analyzed, the other nucleotide chain that does not contain such information is also analyzed. This becomes a factor causing pseudo-results.

Modification of a nucleotide chain is carried out not only for labeling or conjugating, but also for immobilization of the nucleotide chain onto a substrate. A substrate made from nitrocellulose, nylon, or polyvinylidene fluoride, which has either hydrophobicity or positive electric charge, or both properties, has initially been used as the above substrate. Such a substrate, however, has caused dissociation of the immobilized nucleotide chain at times. In recent years, there has been adopted a method, which comprises subjecting the 5'-or 3'-terminus of a nucleotide chain to a modification treatment so as to allow a functional group such as an amino group or aldehyde group to bind thereto, and also previously treating the surface of a substrate such as a glass or silicon so as to convert it into an aldehyde group, epoxy group, or amino group, thereby immobilizing the nucleotide chain onto the substrate. By such a method, a large number of nucleotide chains having different sequences are immobilized per unit area of a substrate, so as to realize a gene analysis microarray. However, such immobilization of a nucleotide chain is also problematic in terms of the aforementioned modification of a nucleotide chain.

### (Patent Documents)

1. Japanese Patent No. 1706289
2. Japanese Patent Laid-Open No. 61-115094
3. Japanese Patent Publication No. 7-31194
4. National Publication of International Patent Application No. 2000-508709
5. Japanese Patent No. 1814713
6. Japanese Patent No. 2093730
7. Japanese Patent No. 2093731
8. Japanese Patent No. 2613877
9. National Publication of International Patent Application No. 10-503841
10. Japanese Paten Application No. 2002-049055
11. Japanese Patent No. 2103155
12. National Publication of International Patent Application No. 2000-502251
13. Japanese Patent Laid-Open No. 11-113599

### (Non-Patent Documents)

1. Chu B. C. F. et al. , Nucleic Acids Res., Vol. 11, p. 6543, 1983
2. Beck. S., Methods Enzymol., Vol. 216, p. 143, 1992
3. Bronstein I. et al., Methods Enzymol., Vol. 217, p. 398, 1993
4. Alves A. M. et al. , Tetrahedron Lett., Vol. 30, p. 3089, 1989
5. Cocuzza A. J. , Tetrahedron Lett., Vol. 30, p. 6287, 1989
6. Theisen P. et al., Tetrahedron Lett., Vol. 33, p. 5033, 1992
7. Kempe T. et al., Nucleic Acids Res., Vol. 13, p. 45, 1985
8. Smith L. M. et al., Nucleic Acids Res., Vol. 13, p. 2399, 1985
9. Ansorge W. et al., Nucleic Acids Res., Vol. 15, p. 4593, 1987
10. Cook A. F., Nucleic Acids Res., Vol. 16, p. 4077, 1988
11. Roget A. et al., Nucleic Acids Res., Vol. 17, 1989
12. Misiura K. et al., Nucleic Acids Res., Vol. 18, p. 4545, 1990
13. Pieles U. et al., Nucleic Acids Res., Vol. 18, p. 4355, 1990
14. Langer P. R. et al., Proc. Natl. Acad. Sci. USA, Vol. 78, p. 6633, 1981
15. Roychoudhury R. et al., Nucleic Acids Res., Vol. 3, p. 101, 1976
16. Vincent C. et al., Nucleic Acids Res., Vol. 10, p. 6787, 1982
17. Yousaf S. I. et al., Gene, Vol. 3, p. 309, 1984
18. Schmitz G. G. et al., Anal. Biochem., Vol. 192, p. 222, 1991
19. Jackson D. A. et al., Proc. Natl. Acad. Sci. USA, Vol. 69, p. 2905, 1972
20. Feinberg A. P. et al., Anal. Biochem., Vol. 132, p. 6, 1983
21. Feinberg A. P. et al., Anal. Biochem., Vol. 137, p. 266, 1984
22. Rigby P. W. J. et al., J. Mol. Biol., Vol. 113, p. 237, 1977
23. Mullis K. B. et al., Methods Enzymol., Vol. 155, p. 335, 1987
24. Saiki R., Science, Vol. 239, p. 487, 1988
25. Siebert P. D. et al., Nature, Vol. 359, p. 557, 1991
26. Day P. J. R. et al., Biochem. J., Vol. 267, p. 119, 1990
27. Schena M. et al., Science, Vol. 270, p. 467, 1992
28. Schalon D. et al., Genome Res., Vol. 6, p. 639, 1996
29. Kakusan Microarray Tokusyu (Featured in Nucleic Acid Microarrays), Nature Genet., Vol. 21, Supplement, January, 1999
30. Amersham Bioscience website:
   (http://www1.amershambiosciences.com/aptrix/upp01077. nsf/Content/microarrays_labelling%5Cmicroarrays_labelling _cyscribe_postlabelling?OpenDocument&hometitle=microarray s), accessed on April 14, 2003
31. Longo M. C. et al., Gene, Vol. 93, p. 125, 1990
32. Thornton C. G. et al., BioTechniques, Vol. 13, p. 180, 1992
33. Sobek H. et al., FEBS Lett., Vol. 388, p. 1, 1996

The present invention has been made to solve the aforementioned problems of the prior art techniques. It is an object of the present invention to provide a method for modifying a nucleotide chain, which is capable of directly modifying the 3'-terminal portion with a modifier, without involving the decomposition of the nucleotide chain, regardless of the base structure of the nucleotide chain.

In addition, it is another object of the present invention to provide a method for modifying a nucleotide chain, which is capable of selectively and easily modifying not only the 3 '-terminus of a single-stranded nucleotide, but also a single chain of a double-stranded nucleotide.

### Disclosure of the Invention

The present invention provides a method for modifying a nucleotide chain, the method comprising:
allowing a nucleotide chain as a target for modification to be degraded by a catabolic enzyme that reacts specifically with a defined base composition, said nucleotide chain including in a 3'-terminal region thereof a nucleotide sequence containing the defined base composition; and
forming a functional group in said nucleotide chain at a 3'-terminal thereof, the functional group being capable of binding a required modifier to the 3'-terminal of said nucleotide chain as a target for modification, wherein hypoxanthine is used as a defined base composition, wherein 3-methyladenine DNA glycosylase is used as a catabolic enzyme and wherein the functional group is an aldehyde group.

In order to achieve the aforementioned objects, the method for modifying a nucleotide chain of the present invention comprises: allowing a catabolic enzyme specific to a nucleotide sequence containing a specific base to act on a nucleotide chain as a target for modification having the above described nucleotide sequence containing a specific base located on the 3'-terminal side thereof; forming a functional group capable of binding to a desired modifier on the 3'-terminus of the above described nucleotide chain as a target for modification; and binding the above described modifier to the 3'-terminus of the above described nucleotide chain having the above described functional group. Using, as a modification target, such a nucleotide chain, on the 3'-terminal side of which the above described nucleotide sequence containing a specific base acting as an enzyme substrate, exists, only the above described nucleotide sequence portion can be decomposed, so as to form a functional group reacting with a desired modifier and binding thereto. By this method, a nucleotide chain can directly be modified with a modifier, thereby easily labeling or conjugating the nucleotide chain. In addition, in a case where immobilization of a nucleotide chain is intended, stable and strong immobilization can be attained using a modifier as a linker.

It is preferable that the nucleotide sequence containing a specif ic base be located on the 3' -terminal side of a nucleotide chain as a host chain, and that the above described specific base does not exist in the above described host chain. With this configuration, when a catabolic enzyme acts on a nucleotide chain as a target for modification, decomposition of a host chain portion can be avoided. Thus, it becomes possible to maintain a state where the sequence information of a host chain portion is completely conserved. In order to determine the sequence information of a nucleotide chain, the nucleotide chain preferably has a nucleotide sequence consisting of approximately at least 10 bases.

The nucleotide sequence containing a specific base may be added to the 3'-terminal side of the nucleotide chain as a host chain. This is a method of deliberately adding nucleotides having bases acting as enzyme substrates to the nucleotide chain as a target for modification. Regardless of whether or not the nucleotide chain as a target for modification is a single strand or a double strand, it is possible to specifically modify the 3'-terminus of the nucleotide chain simply with a modifier. By appropriately selecting a nucleotide sequence to be added, the uptake of an unnecessary nucleotide sequence is eliminated.

More specifically, a nucleotide chain having, in the 3'-terminal region thereof, a sequence complementary to the 3'-terminal region of a single-stranded nucleotide to be modified is annealed to the above described single-stranded nucleotide to be modified, so that a complementary nucleotide sequence containing a specific base may be added to the 3'-terminal side of the above described single-stranded nucleotide to be modified.

In addition, a double-stranded nucleotide, at least one nucleotide chain of which is to be modified, may be cleaved with a restriction enzyme that specifically recognizes a nucleotide sequence in the 3'-terminal region of the nucleotide chain as a target for modification, and a complementary nucleotide sequence containing a specific base may be added to the 3'-terminal side of the cleaved nucleotide chain as a target for modification.

Moreover, a double-stranded nucleotide having a nucleotide sequence containing a specific base in the 5'-terminal region thereof is ligated to the 3'-terminal side of a nucleotide chain as a target for modification, which is at least one nucleotide chain of a double-stranded nucleotide, using a DNA ligase, and the ligated nucleotide chain is then cleaved with a restriction enzyme that specifically recognize a nucleotide sequence ranging from the above described specific base exclusive to the 3'-terminus thereof, so that the nucleotide sequence containing a specific base may be added to the 3'-terminal side of the above described nucleotide chain as a target for modification.

Addition of a nucleotide sequence may be carried out by incorporation of nucleotides into a nucleotide chain as a target for modification using a DNA polymerase.

The nucleotide chain having the nucleotide sequence containing a specific base may be a chemically synthesized nucleotide chain.

Preferably, an oligonucleotide having a nucleotide sequence complementary to the specific base is added before a catabolic enzyme is allowed to act. By allowing a complementary bond to generate between the bases of the two nucleotide chains, the substrate specificity of enzymes can be increased, thereby improving reaction efficiency.

An aldehyde group may be formed on the 3'-terminus of a nucleotide chain as a target for modification, for example. Specifically, an aldehyde group may be formed on the 3'-terminus of a nucleotide chain as a target for modification, using a DNA glycosylase as a catabolic enzyme. More specifically, when the specific base is hypoxanthine, an aldehyde group may be formed on the 3'-terminus of a nucleotide chain as a target for modification, using 3-methyladenine DNA glycosylase as a catabolic enzyme.

There may be various combinations of specific bases with catabolic enzymes. However, since incorporation of uracil into a nucleotide chain has widely been used in various methods for gene analysis, there are some cases where the use of uracil as such a specific base is not desired. Using hypoxanthine that has not frequently been used and a catabolic enzyme thereof, it becomes possible to significantly improve convenience and user-friendliness in modification of the 3'-terminus of a nucleotide chain. In addition, regardless of the structure of bases of a nucleotide chain or a chain length, it becomes possible to quantitatively and stably modify the 3'-terminal side of the nucleotide chain, directly and simply with any given modifier. This is a method capable of specifically modifying the 3'-terminus of a nucleotide chain, while preventing incorporation of an unnecessary nucleotide sequence or modified bases into the nucleotide chain.

In addition to the aforementioned combination of hypoxanthine with 3-methyladenine DNA glycosylase, an aldehyde group may also be formed on the 3'-terminus of a nucleotide chain as a target for modification using the combinations shown in the following Table 1, for example.

**Table 1**

| Incorporated base composition in 3'-terminus of nucleotide chain | Applied DNA glycosylase/ DNA repair enzyme |
|---|---|
| · Hypoxanthine | · Hypoxanthine DNA |
| | glycosylase |
| · 3-methyladenine | · 3-methyladenine DNA |
| · 3-ethyladenine | glycosylase type I |
| · 3-methyladenine | · 3-methyladenine DNA |
| · Hypoxanthine | glycosylase type II |
| · Xanthine | |
| · 3-methylguanine | |
| · 7-methylguanine | |
| · 1,N⁶-ethanoadenine | |
| · 8-oxoguanine | |
| · 7-ethylpurine | |
| · 3-ethylpurine | |
| · 7,3-diethylpurine | |
| ·1-carboxyethyladenine | |
| · 7-carboxyethylguanine | |
| · O²-methylpyrimidine | |
| · 7-(2-ethoxyethyl)guanine | |
| · 7-(2-hydroxyethyl)guanine | |
| · 7-(2-chloroethyl)guanine | |
| · 1,2-bis(7-guanyl)ethane | |
| · 3-ethylthioethylpurine | |
| · N^{2,3}-ethanoguanine | |
| · N^{2,3}-ethenoguanine | |
| · 5-hydroxymethyluracil | |
| · 5-formyluracil | |
| · 1, N⁴-ethenocytosine | |
| ·1,N²-ethenoguanine | |
| · 3,N²-ethenoguanine | |
| · Uracil | · Uracil DNA glycosylase |
| · 5-hydroxyuracil | |
| · 5,6-dihydroxyuracil | |
| · 5-fluorouracil | |
| · Thymine glycol | · Endonuclease III |
| · 5,6-dihydrothymine | |
| · 5,6-dihydroxydihydrothymine | |
| · Hydroxycytosine | |
| · Urea | |
| · 5-hydroxy-5-methylhydantoin | |
| · 6-hydroxy-5, | |
| · 6-dihydroxypyrimidine | |
| · 5-hydroxyuracil | |
| · 5-hydroxy-6-hydrothymine | |
| · 5,6-dihydrouracil | |
| · Uracil glycol | |
| · 5-hydroxy-6-hydrouracil | |
| · 8-oxoguanine | · 8-oxoguanine DNA |
| · 7,8-dihydro-8-oxoguanine | glycosylase |
| · 8-oxoadenine | |
| · Formamide guanine | |
| · Methylformamide guanine | |
| · 7-methylguanine | · Formamide pyrimidine DNA |
| · 1,6-diamino-5-formamide | glycosylase |
| pyrimidine | |
| · Formamide guanine | |
| · Methylformamide guanine | |
| · Formamide adenine | |
| · 8-oxoadenine | |
| · 8-oxoguanine | |
| · 7,8-dihydro-8-oxoguanine | |
| · Hydroxycytosine | |
| · 5-hydroxyuracil | |
| · Aflatoxin-bound imidazole | |
| ring-opened guanine | |
| · Imidazole ring-opened | |
| N-2-aminofluorene-C8-guanine | |
| · 8-oxoguanine | · Mut Y-DNA glycosylase |
| · Adenine/guanine mismatch | |
| · 1,N⁴-ethenocytosine | · Mismatch uracil DNA |
| · Uracil/guanine mismatch | glycosylase |
| · Pyrimidine dimmer | · Pyrimidine dimmer DNA |
| | glycosylase |
| · Thymine/guanine mismatch | · Thymine DNA glycosylase |
| · Guanine/guanine mismatch | |

A modifier having an amino group that can bind to a functional group formed on the 3'-terminus of a nucleotide chain can preferably be used herein.

Such a modifier may be a substance for labeling or conjugating a nucleotide chain. With such a configuration, the modified nucleotide chain can be used as a detection probe or target in gene analysis.

Preferred examples of such a modifier used herein may include a fluorescent substance, vitamin, a lipid, amino acid, oligopeptide, a protein, and an exogenous substance. Of these, amino acid, oligopeptide, and a protein have an amino group. With regard to other components, if an amino group is given to them, these components may be fused with an aldehyde group existing at the 3'-terminus of a nucleotide chain. Thus, the functions of a fluorescent substance, vitamin, a lipid, amino acid, oligopeptide, a protein, or an exogenous substance may be added to the nucleotide chain.

Such a modifier may be a substance capable of binding to a substrate used for gene analysis. With such a configuration, the modified nucleotide chain can stably be immobilized as a detection probe or target on a substrate.

An aminoalkanethiol or amino silane coupling agent can preferably be used as such a modifier. These compounds can stably bind to a substrate made from noble metal, glass, or resin.

### Brief Description of the Drawings

FIG. 1 is a view showing the principle of the method for modifying a nucleotide chain in Embodiment 1;
FIG. 2 is a chemical equation showing a part of steps of the method for modifying a nucleotide chain shown in FIG. 1, which is a step of modifying the generated aldehyde derivative with fluorescein;
FIG. 3 is a chemical equation showing a part of steps of the method for modifying a nucleotide chain shown in FIG. 1, which are a step of modifying the generated aldehyde derivative with alkanethiol and a step of immobilizing the derivative on a noble metal substrate;
FIG. 4 is a view showing the principle of the method for modifying a nucleotide chain in Embodiment 2;
FIG. 5 is a chemical equation of the reaction center of the nucleotide chain shown in FIG. 4;
FIG. 6 is a view showing the principle of the method for modifying a nucleotide chain in Embodiment 3;
FIG. 7 is a view showing the principle of the method for modifying a nucleotide chain in Embodiment 4;
FIG. 8 is a view showing the principle of the method for modifying a nucleotide chain in Embodiment 5;
FIG. 9 shows the results of polyacrylamide gel electrophoresis performed on the nucleotide chain in each reaction step of the method for modifying a nucleotide chain in Example 1 of the present invention;
FIG. 10 shows the results of film exposure performed on the nucleotide chain in each reaction step of the method for modifying a nucleotide chain in Example 2 of the present invention; and
FIG. 11 shows the results of polyacrylamide gel electrophoresis and film exposure performed on the nucleotide chain in each reaction step of the method for modifying a nucleotide chain in Example 3 of the present invention.

### Best Mode for Carrying Out the Invention

The embodiments will be described below, referring to the drawings.

### (Embodiment 1)

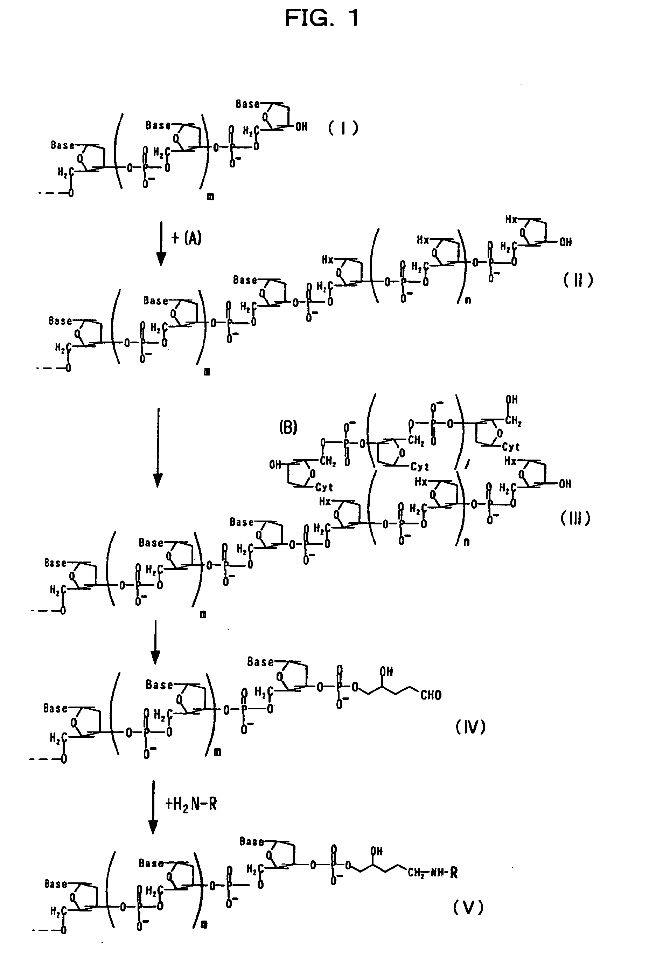

Embodiment 1 of the present invention will be described based on FIG. 1. In the figure, each of l, m, and n represents null or any given natural number. The term "Base" represents any given base such as adenine, guanine, cytosine, thymine, uracil, or the like. Hx represents hypoxanthine, and Cyt represents cytosine.

Terminal deoxynucleotidyltransferase is allowed to act on a nucleotide chain (I) having any given nucleotide sequence and 2'-deoxyinosine-5'-triphosphate (A) (which has hypoxanthine Hx, as shown in the structure indicated below), so as to add a nucleotide sequence including hypoxanthine to the 3'-terminus of the nucleotide chain (I), thereby obtaining a nucleotide chain (II).

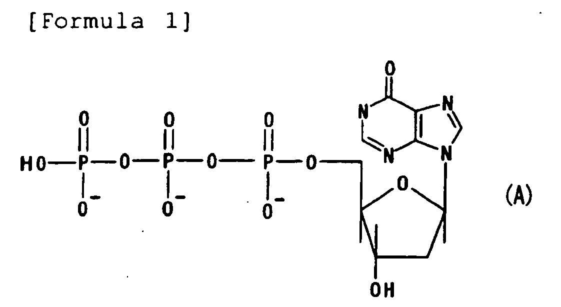

An oligonucleotide chain (B) consisting of several dozens of nucleotides including cytosine is annealed to the obtained nucleotide chain (II), so as to obtain a nucleotide chain (III).

Thereafter, 3-methyladenine DNA glycosylase II is allowed to act on the nucleotide chain (III) a portion of which has become a double strand. The resultant is then subjected to an alkaline heat treatment, so as to cleave a glycosidic bond of nucleotides including hypoxanthine. The remaining deoxyribose-phosphate bond is then cleaved, so as to obtain a nucleotide chain (IV) having an aldehyde group at the 3'-end thereof.

Thereafter, a modifier having an amino group (H₂N-R (which will be described later)) is allowed to react with the nucleotide chain (IV), so as to obtain a Schiff base formed by a dehydration condensation reaction of the nucleotide chain (IV) and the modifier, that is, a nucleotide chain (V) the 3'-end of which has directly been modified with the modifier.

If a nucleotide chain of interest has a length consisting of several dozens of nucleotides that can be chemically synthesized, it is possible to omit the first reaction by previously synthesizing a nucleotide chain having a specific base.

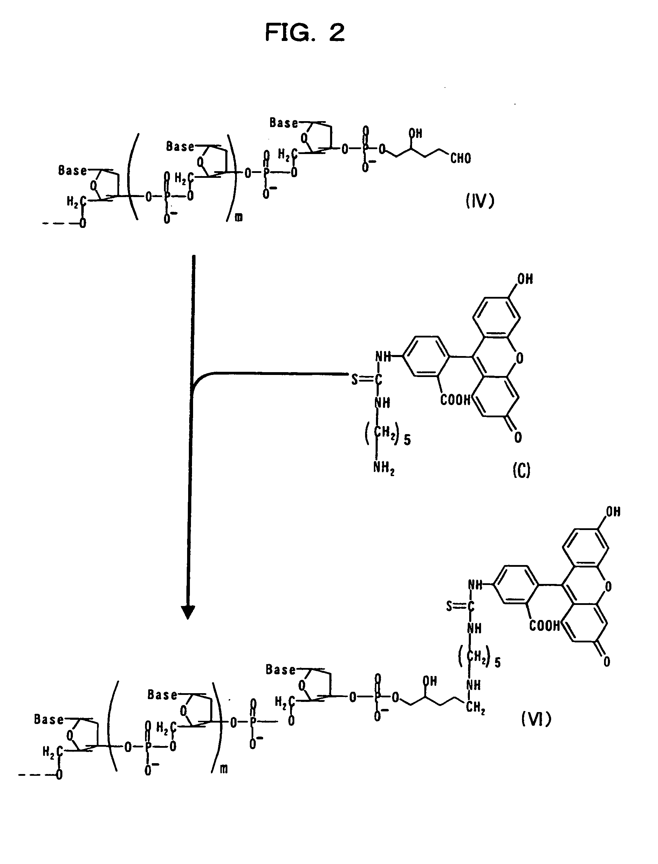

As shown in FIG. 2, fluorescein cadaverine (C), which has been known as a fluorescent substance, is allowed to react with the nucleotide chain (IV) having an aldehyde group in the 3'-end thereof, so as to obtain a nucleotide chain (VI), to the 3'-end of which fluorescein cadaverine has directly bound.

Thus, by modifying a nucleotide chain with fluorescein, the nucleotide chain can be labeled (conjugated) with fluorescence. This nucleotide chain can be used as a probe or target for gene analysis such as hybridization.

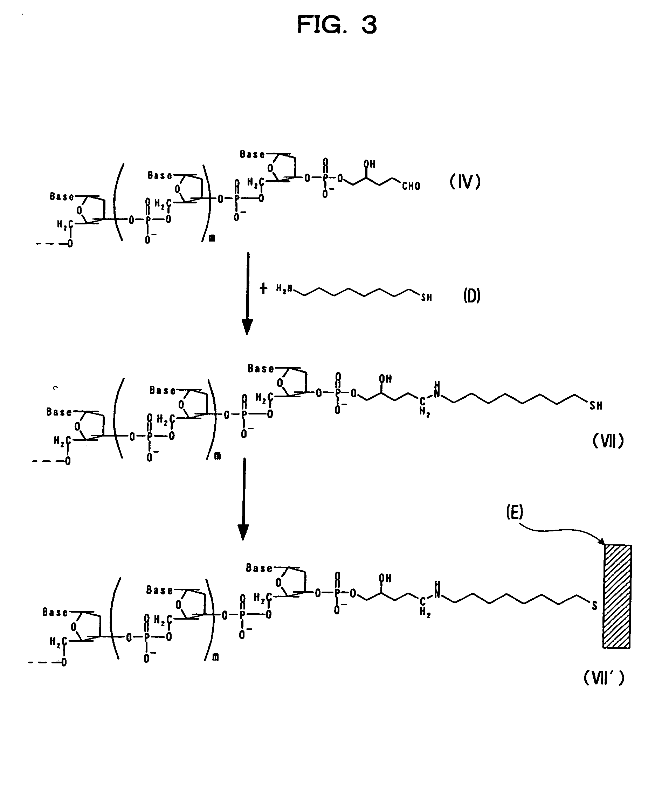

As shown in FIG. 3, thiol compound having an amino group, and specifically herein 8-amino-1-octanethiol (D), is allowed to react with the nucleotide chain (IV) having an aldehyde group in the 3'-end thereof, so as to obtain a modified nucleotide chain (VII), to the 3'-end of which an 8-amino-1-octanethiol residue has directly bound.

This modified nucleotide chain (VII) is allowed to act on a noble metal substrate (E), so as to form a strong covalent bond between a thiol group and the substrate (E), thereby immobilizing a nucleotide chain (VII') onto the substrate (E).

Thus, by immobilizing a nucleotide chain onto the substrate (E), measurement methods using noble metal as a substrate, such as electrochemical measurement, quartz crystal oscillator microbalance, or surface plasmon resonance, can be applied to gene analysis.

A modifier for labeling or conjugating a nucleotide chain may appropriately have ability to bind to a functional group that is formed on the 3'-terminus of the nucleotide chain. For example, in the case of fluorescent substances such as fluorescein, Texas Red, rhodamine and cyanine compounds such as Cy3 or Cy5 as typical examples, and non-fluorescent substances such as biotin or digoxigenin, by forming an amino group at the 3'-terminus thereof, these substances can be used as modifiers. A nucleotide chain labeled or conjugated with these modifiers can be used as a probe or target for gene analysis such as hybridization.

Amino acid, a peptide, and a protein may also be used as modifiers. Among amino acids, since phenylalanine, tryptophan, and tyrosine have fluorescent properties, these amino acids can be used for labeling or conjugating. Other amino acids or peptides that have no fluorescent properties can also be used for labeling or conjugating by allowing fluorescein or the like (hereinafter referred to as a labeling compound) to bind to the side chain thereof.

A peptide formed with two or more amino acids linkage has side chains corresponding to the number of the amino acids. Thus, it is possible to allow a plurality of labeling compounds to bind to such a peptide. For example, since trilysine has 3 amino acids at the side chains thereof, it is possible to allow at maximum 3 labeling compounds having a carboxyl group, a succinimide group, or the like to bind to the peptide.

Not only peptides, but also substances formed by modifying alkyl compounds, allyl(compounds), cycloalkane(compounds), aromatic(compounds), or sugars such as hydrocargone having an amino group, with labeling compounds, are used as modifiers. A nucleotide chain can also be modified with the thus obtained modifiers, thereby realizing labeling or conjugating of a nucleotide chain.

Moreover, alkaline phosphatase, peroxidase, transferrin having color and fluorescent properties, hemoglobin, and proteins such as a green fluorescent protein, a blue fluorescent protein, or aequorin, are used as modifiers, so as to label or conjugate a nucleotide chain. These modifiers are useful for gene analysis, and particularly for *in situ* hybridization.

Furthermore, noble metal colloids, such as gold colloids or silver colloids, on the surface of which an amino group is formed, fine particles including, as typical examples, fine magnetic particles and fine polymer particles such as polystyrene beads, can also be used as modifiers for nucleotide chains.

When a nucleotide chain is immobilized on noble metal, among thiol compounds that are generically called alkanethiol, those having an amino group can be adopted as modifiers. Since a nucleotide chain can be immobilized on noble metal via a modifier, measurement methods using noble metal as a substrate, such as electrochemical measurement, quartz crystal oscillator microbalance, or surface plasmon resonance, can be applied to gene analysis.

The structure of alkanethiol used herein is not particularly limited, as long as it contains a thiol group that binds to noble metals and an amino group. A hydrocarbon residue existing between an amino group and a thiol group may be in various forms such as a linear, branched, cycloring, allyl chain, or aromatic form. The number of carbons, the position of an amino group, and the like may also vary.

A gold substrate is generally used in quartz crystal oscillator microbalance, surface plasmon resonance, etc. Depending on the measurement method applied, platinum, silver, copper palladium, indium, nickel, iron, aluminum, and alloy thereof may also be used as substrates.

A silane coupling agent having an amino group may also be used as a modifier. With such a silane coupling agent, a nucleotide chain can be immobilized onto a substrate such as a glass, silicon, silica, alumina, mica, or polymer resin such as polystyrene, nylon, or epoxy. It can get applied to various conventional gene analysis methods.

As with alkanethiol, the structure of a silane coupling agent is not particularly limited, as long as it is a compound having an amino group and an alkoxy group. Examples of a silane coupling agent used herein may include
γ-aminopropyltriethoxysilane,
N-β(aminoethyl)-γ-aminopropylmethyldimethoxysilane,
N-β(aminoethyl)-γ-aminopropyltrimethoxysilane,
N-β(aminoethyl)-γ-aminopropyltriethoxysilane, and
γ-aminopropyltrimethoxysilane.

Substances that do not originally have an amino group can also modify a nucleotide chain through the mediation of an appropriate spacer having an amino group. For example, a substance having a carboxyl group can modify a nucleotide chain through the mediation of a substance having a diamino structure, such as 1,2-diaminoethane or 1,6-diaminohexane, as a spacer

Moreover, substances having a hydrazine group, an aminoxyl group, and a cyano group, or substances having halogenated magnesium, such as Grignard reagent, may also be used as modifiers.

### (Embodiment 2)

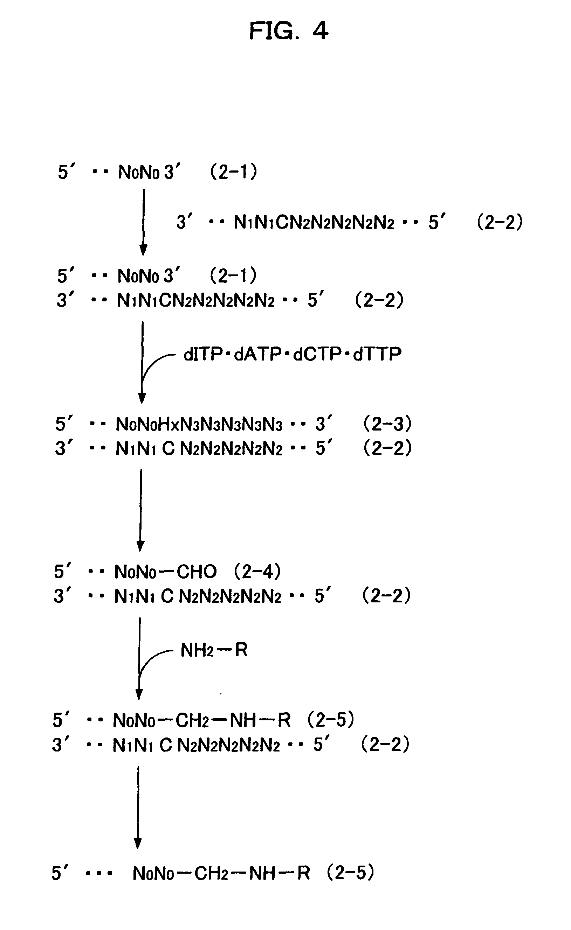

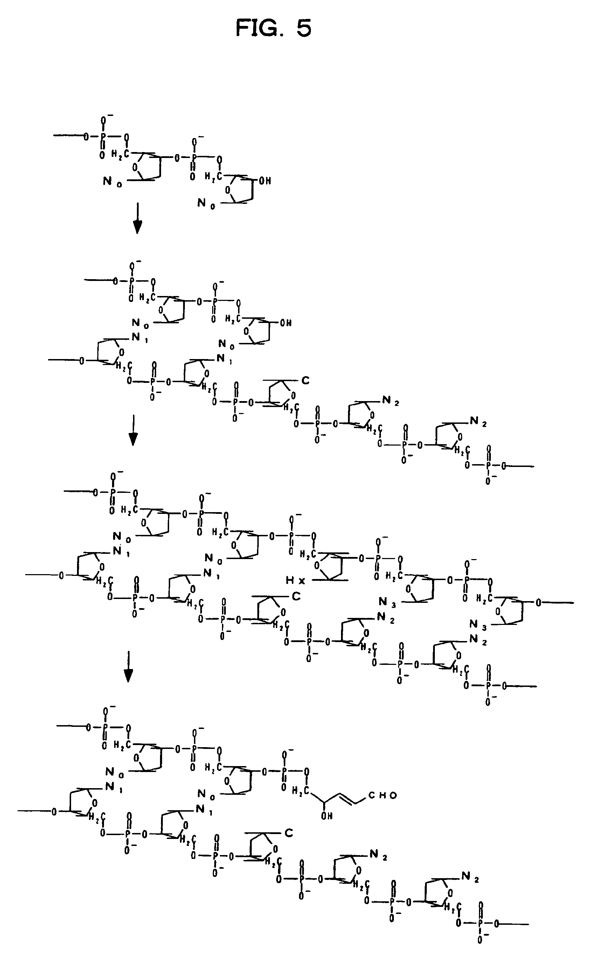

FIG. 4 shows the principle of the method for modifying a nucleotide chain in Embodiment 2. FIG. 5 shows a chemical equation of the reaction center of the nucleotide chain shown in FIG. 4.

In FIGS. 4 and 5, No represents any base selected from the group consisting of adenine, guanine, thymine, and cytosine. N₁ represents any base selected from the group consisting of adenine, guanine, thymine, and cytosine, which forms a complementary base pair with No. N₂ represents any base selected from the group consisting of adenine, guanine, thymine, and cytosine, which does not form a complementary base pair with N₀. N₃ represents any base selected from the group consisting of adenine, hypoxanthine, thymine, and cytosine, which forms a complementary base pair with N₂. C represents cytosine, and Hx represents hypoxanthine. All of these N₀, N₁, N₂, N₃, C, and Hx are included in 2'-deoxynucleotide (hereinafter referred to simply as nucleotide). With regard to N₀, N₁, N₂, and N₃, only the meanings thereof are indicated. The number thereof is not intended to be limited.

Furthermore, dITP, dATP, dCTP, and dTTP represent
2'-deoxyinosine-5'-triphosphate,
2'-deoxyadenosine-5'-triphosphate,
2'-deoxycytidine-5'-triphosphate, and 2'-deoxytymidine-5'-triphosphate, respectively. NH₂-R represents any given modifier having an amino group, as described above.

A nucleotide chain as a target for modification (2-1: corresponds to the number shown in FIG. 4; hereinafter such numbers have the same meanings as the above-mentioned) has the sequence of base No in the 3'-terminal region thereof. Another nucleotide chain (2-2) is annealed to this nucleotide chain (2-1), so as to form a double-stranded nucleotide. The nucleotide chain (2-2) has, on the 3'-terminal region thereof, the sequence of any given base N₁ that is complementary to base No in the 3'-terminal region of the nucleotide chain (2-1). The nucleotide chain (2-2) also has cytosine C that islocated adjacent to the base N₁ in the 5'-terminal region, and further has the sequence of any given base N₂ that is not complementary to the sequence of the base No. After completion of the annealing, the 5'-terminal region protrudes.

This partial double-stranded nucleotide is allowed to incorporate dITP, dATP, dCTP, and dTTP, using DNA polymerase as a catalyst, so as to form a complete double-stranded nucleotide. The thus formed nucleotide chain (2-3) is formed by adding a nucleotide sequence that is complementary to the single-stranded region of the nucleotide chain (2-2) to the 3'-terminal region of the nucleotide chain (2-1). The nucleotide chain (2-3) has hypoxanthine Hx that corresponds to cytosine C of the nucleotide chain (2-2) as complementary base pair.

Thereafter, 3-methyladenine DNA glycosylase II is allowed to act on the thus formed complete double-stranded nucleotide. The resultant is then applied to an alkaline heat treatment, so as to specifically decompose a glycosidic bond existing between a carbon at 1'-position and oxygen of nucleotide (deoxyribose) including hypoxanthine Hx. An aldehyde group is then formed on the 3'-end of a nucleotide chain (2-4) formed by the above decomposition.

Subsequently, a modifier (NH₂-R) having an amino group is added, and each aldehyde group is thereby allowed to react with each amino group, so as to form a Schiff base. The thus formed nucleotide chain (2-5) is a product of interest, wherein a modifier directly binds to the 3'-end of the nucleotide chain (2-1) as a starting substance.

After completion of the modification, the unnecessary nucleotide chain (2-2) is separated and removed. Such removal of the nucleotide chain (2-2) is easily carried out by subjecting the double-stranded nucleotide, to which a modifier binds, to a heat treatment, or by adding a phosphate group to the 5'-end of the nucleotide chain (2-2) and then allowing lambda exonuclease that specifically decomposes it to act thereon. By previously substituting a portion of guanines of bases N₁ and N₂ of the nucleotide chain (2-2) with hypoxanthines, the nucleotide chain can also be fragmented with 3-methyladenine DNA glycosylase II.

### (Embodiment 3)

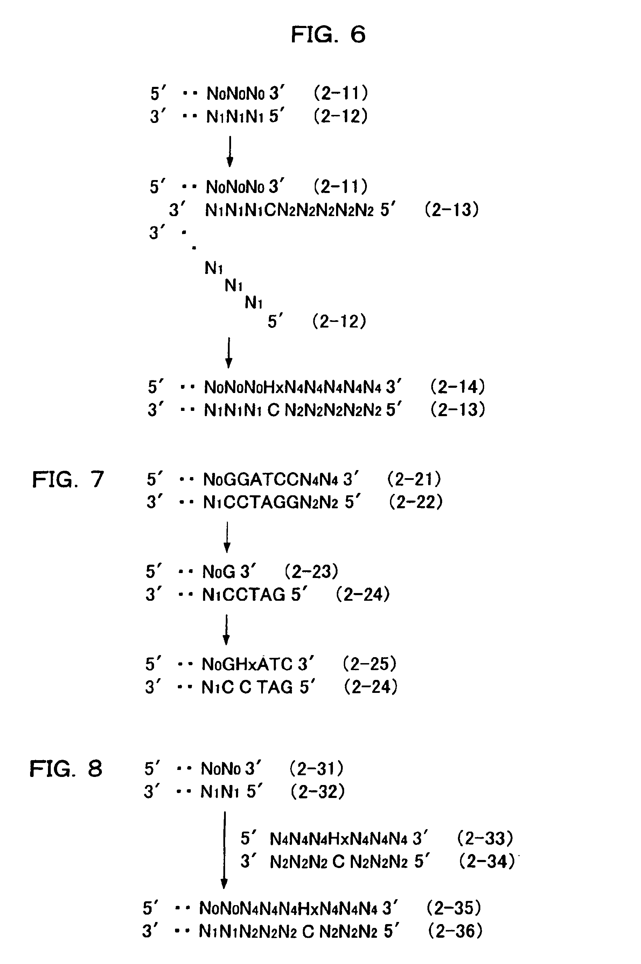

FIG. 6 shows the principle of the method for modifying a nucleotide chain in Embodiment 3. This method comprises addition of a nucleotide sequence including hypoxanthine acting as a substrate to the aforementioned 3-methyladenine DNA glycosylase II, to a nucleotide chain as a target for modification out of a double-stranded nucleotide.

In the figure, N₀, N₁, N₂, C, Hx, 5', and 3' have the same meanings as described above. N₄ represents any base selected from the group consisting of adenine, guanine, thymine, and cytosine, which forms a complementary base pair with N₂. A represents adenine, G represents guanine, and T represents thymine, respectively.

The double-stranded nucleotide consists of a first nucleotide chain (2-11) and a second nucleotide chain (2-12). Of these, the nucleotide chain (2-11) is a target for modification. The nucleotide chain (2-11) has the sequence of base No in the 3'-terminal region thereof. The nucleotide chain (2-12) has the sequence of base N₁ in the 5'-terminal region thereof.

This double-stranded nucleotide is applied to a heat treatment or an alkaline treatment, so as to dissociate it into a single strand. A third nucleotide chain (2-13) is then annealed to the dissociated nucleotide chain (2-11), so as to form a partial double-stranded nucleotide. The third nucleotide chain (2-13) has, on the 3'-terminal side thereof, the sequence of any given base N₁ that is complementary to base No in the 3'-terminal region of the nucleotide chain (2-11). The nucleotide chain (2-13) also has cytosine C that is located adjacent to the base N₁ in the 5'-terminal region, and further has the sequence of any given base N₂ that is not complementary to the sequence of the base No. After completion of the annealing, a portion from cytosine C to the 5'-terminus becomes a single strand.

This partial double-stranded nucleotide is allowed to incorporate dITP, dATP, dCTP, and dTTP, using DNA polymerase as a catalyst, so as to form a complete double-stranded nucleotide. The thus formed nucleotide chain (2-14) is formed by adding a nucleotide sequence that is complementary to the single-stranded region of the nucleotide chain (2-2) to the 3'-terminal of the nucleotide chain (2-11). The nucleotide chain (2-14) has hypoxanthine Hx at the position corresponding to cytosine C of the nucleotide chain (2-2).

By performing the same operations as those described in Embodiment 2 on this complete double-stranded nucleotide, an aldehyde group can be formed on the 3'-end of the nucleotide chain (2-11), a modifier can be allowed to bind thereto, and an unnecessary nucleotide chain can be removed.

A method for modifying only an either single nucleotide chain as a target in double strand is exemplified herein. However, it could be understood that it is also possible to modify both the nucleotide chains in the same above manner.

### (Embodiment 4)

FIG. 7 shows the principle of the method for modifying a nucleotide chain in Embodiment 4. This is a method for modifying a nucleotide chain, which is applied when an appropriate restriction site is found in a double-stranded nucleotide.

In the figure, N₀, N₁, A, G, T, C, and Hx have the same meanings as described above. N₂ and N₄ represent any given bases selected from the group consisting of adenine, guanine, thymine, cytosine, hypoxanthine, and uracil, which are complementary to each other.

The double-stranded nucleotide consists of a first nucleotide chain (2-21) and a second nucleotide chain (2-22). Of these, the nucleotide chain (2-21) is a target for modification. A nucleotide sequence (GGATCC/CCTAGG) that is recognized and cleaved with restriction enzyme BamHI is found in the 3'-terminal region of the nucleotide chain (2-21) (in the 5'-terminal region of the nucleotide chain (2-22)).

First, the double-stranded nucleotide is cleaved with restriction enzyme BamHI, so as to form a partial double-stranded nucleotide consisting of a nucleotide (2-23) wherein only G is found in the 3'-end of N₀, and a nucleotide (2-24) wherein 3'CCTAG5' is found in the 5'-terminal region of N₁.

This partial double-stranded nucleotide is allowed to incorporate dITP, dATP, dCTP, and dTTP, using DNA polymerase as a catalyst, so as to form a complete double-stranded nucleotide. The thus formed nucleotide chain (2-25) is formed by adding a nucleotide sequence that is complementary to the single-stranded region of the nucleotide chain (2-24) to the 3'-end of the nucleotide chain (2-23). The nucleotide chain (2-25) has hypoxanthine Hx at the position corresponding to cytosine C of the nucleotide chain (2-24).

By performing the same operations as those described in Embodiment 2 on this complete double-stranded nucleotide, only 1 nucleotide can be added to the 3'-end of the nucleotide chain (2-21), and an aldehyde group can be added thereto. A modifier can be then allowed to bind thereto, and an unnecessary nucleotide chain can be removed.

### (Embodiment 5)

FIG. 8 shows the principle of the method for modifying a nucleotide chain in Embodiment 5. In this method, double-stranded nucleotide chains that have previously incorporated hypoxanthine into their nucleotide sequences are ligated to each other.

In the figure, N₀, N₁, C, and Hx have the same meanings as described above. N₂ and N₄ represent any given bases selected from the group consisting of adenine, guanine, thymine, cytosine, hypoxanthine, and uracil, which are complementary to each other.

The double-stranded nucleotide consists of a first nucleotide chain (2-31) and a second nucleotide chain (2-32). Of these, the nucleotide chain (2-31) is a target for modification. The nucleotide chain (2-31) has the sequence of base No in the 3'-terminal region thereof. The nucleotide chain (2-32) has the sequence of base N₁ that forms a complementary base pair with the 3'-terminal region of the nucleotide chain (2-31) in the 5'-terminal side thereof.

Another double-stranded nucleotide is ligated to the above double-stranded nucleotide using DNA ligase as a catalyst. Another double-stranded nucleotide consists of a nucleotide chain (2-33) that has the sequence of base N₄ on both sides of hypoxanthine Hx and a nucleotide chain (2-34) that has base N₂ complementary to base N₄ on both side of cytosine C. The two double-stranded nucleotide chains are ligated to each other, such that the nucleotide chain (2-33) can be located on the 3'-terminal side of the nucleotide chain (2-31) to be modified. By such ligation, a double-stranded nucleotide consisting of a nucleotide chain (2-35) (the nucleotide chain (2-31) and the nucleotide chain (2-33)) and a nucleotide chain (2-36) (the nucleotide chain (2-31) and the nucleotide chain (2-33)) is formed.

By performing the same operations as those described in Embodiment 2 on this complete double-stranded nucleotide, an aldehyde group can be formed on the 3'-terminus that is located close to the No sequence of the nucleotide chain (2-31), a modifier can be allowed to bind thereto, and an unnecessary nucleotide chain can be removed.

In Embodiment 5, a nucleotide sequence consisting of 3 base pairs (N₂-N₄) is allowed to exist on a portion from hypoxanthine Hx to the 5'-terminus (and from cytosine C to the 3'-terminus). This nucleotide sequence portion can be omitted.

However, since formation of a sequence recognized with restriction enzymes at the ligation site results in a high efficiency in the ligation reaction with DNA ligase, it is preferable that a nucleotide sequence consisting of 3 or 4 base pairs be allowed to exist on the portion from hypoxanthine Hx to the 5'-terminus (and from cytosine C to the 3'-terminus).

For example, when the base No sequence at the 3'-terminus of the nucleotide chain (2-31) and the base N₁ sequence at the 5'-terminus of the nucleotide chain (2-32) are those shown in the sequence (I) indicated below, if a sequence portion from hypoxanthine Hx to the 5'-terminus in the nucleotide chain (2-33) and a sequence portion from cytosine C to the 3'-terminus in the nucleotide chain (2-34) are allowed to have the sequence (II) indicated below, the sequence (III) indicated below is formed at the ligation site. Since this sequence (III) is a sequence portion that is recognized and cleaved with restriction enzyme HincII, the ligase reaction efficiently progresses.

| | | |
|---|---|---|
| ..GTC 3' | 5' GAC.. | ..GTCGAC.. |
| ..CAG 5' | 3' CTG.. | ..CAGCTG.. |
| (I) | (II) | (III) |

The termini of the two double-stranded nucleotide chains to be ligated may be either blunt ends as in the case of the aforementioned nucleotide chain, or protruding ends wherein only a single nucleotide chain protrudes, as with a site cleaved with BamHI.

Cleavage of a nucleotide sequence with restriction enzyme and ligation thereof using DNA ligase have conventionally been used for incorporation of a gene into a vector. By the combined use of these operations with the method, incorporation of a gene (nucleotide chain) into a vector and modification with a modifier can be carried out under the same reaction conditions. Thus, a valuable nucleotide chain sample obtained by amplification of a trace amount of analyte according to PCR or the like can effectively be used without wasting it. For example, a pET vector (Studier F. et al., Methods Enzymol., Vol. 185, p. 60) that is a gene expression vector has a BamHI site at a gene insertion site thereof. Accordingly, by applying the method described in Embodiment 4, modification of a nucleotide chain and incorporation thereof into the vector can simultaneously be realized.

The length of a nucleotide chain as a target for modification may be at least 3 nucleotides. However, when such a nucleotide chain is used for gene analysis, the length of the used nucleotide chain should be determined. In this case, the length is preferably at least 10 nucleotides.

When the methods described in Embodiments 2 and 3 are applied to a nucleotide sequence corresponding to a portion from hypoxanthine Hx to the 3'-terminus, the nucleotide sequence may have a chain length consisting of at least one base pair. However, when the methods described in Embodiments 4 and 5 are applied to the same above nucleotide sequence, it preferably has a chain length consisting of 2 to 4 base pairs or more. In order to allow restriction enzyme or ligase to sufficiently act on a nucleotide chain, the nucleotide chain should have a length that is somewhat longer than that of a sequence portion recognized with each enzyme.

In each of the above described embodiments, addition of a nucleotide sequence including hypoxanthine Hx, or action of 3-methyladenine DNA glycosylase II having, as a substrate, nucleotide including hypoxanthine, are exemplified. However, examples are not limited thereto. When nucleotides including the bases shown in Table 1 (as shown above) in the 3'-terminal region thereof are used together with DNA glycosylase as well, the 3'-terminus of the nucleotide chain can be modified with a desired modifier.

The present invention will be illustrated in more detail in the following specific examples.

### (Example 1)

A chemically synthesized single-stranded nucleotide chain consisting of 30 nucleotides (procured from Sigma-Genosys) was used as a nucleotide chain as a target for modification. This nucleotide chain encodes positions 8 to 37 of the 16S ribosome ribonucleic acid gene of *Escherichia coli*. It has the nucleotide sequence indicated below from the 5'-terminus. A, T, G, and C represent nucleotides in deoxynucleotide, adenine, thymine, guanine, and cytosine, respectively.
AGAGTTTGATCATGGCTCAGATTGAACGCT (Sequence 1)

First, the nucleotide chain was mixed into 50 µl of a reaction solution consisting of 2 units (hereinafter abbreviated as u)/µl of terminal deoxynucleotidyltransferase (manufactured by Invitrogen), 10 mM 2'-deoxyinosine-5'-triphosphate, 2 mM cobalt chloride, 1 mM dithiothreitol, and 0.1 M potassium cacodylate (pH 7.2), thereby resulting in the concentration of the above nucleotide chain of 5 µM. Thereafter, the mixture was reacted at 37°C for 4 hours, so as to tail a nucleotide sequence including hypoxanthine to the 3'-terminal side of the nucleotide chain.

This nucleotide chain was recovered by ethanol precipitation. 100 µM oligodeoxycytosine (the number of nucleotides: 18; manufactured by New England BioLabs) was then added thereto, so that it was annealed to the hypoxanthine in the nucleotide sequence that had been tailed to the 3'-terminal side of the nucleotide chain.

Subsequently, the nucleotide chain was mixed into a reaction solution consisting of 0.3 u/µl 3-methyladenine DNA glycosylase type II (manufactured by Trevigen), 1 mM ethylenediaminetetraacetic acid, 1 mM glycoletherdiaminetetraacetic acid, 1 mM dithiothreitol, and 10 mM 2-[4-(2-hydroxyethyl)-1-piperazinyl]ethanesulfonic acid-potassium hydroxide (pH 7.4). The obtained mixture was reacted at 37°C for a day and a night, so as to cleave a glycosidic bond of the nucleotide sequence that had been tailed to the 3'-terminal side of the nucleotide chain.

Sodium hydroxide was added to this reaction solution to a concentration of 0.1 M. The mixture was heated at 100°C for 5 minutes, so as to dissociate the phosphate group remaining at the 3'-terminal side of the nucleotide chain and form an aldehyde group in the 3'-end.

This nucleotide chain was recovered by ethanol precipitation, and it was then mixed with 5 mM fluorescein cadaverine (manufactured by Molecular Probes) under conditions of 0.1 M sodium carbonate (pH 9.5). Thereafter, 50 µl of the reaction solution was reacted at a room temperature for 2 hours, so as to form a Schiff base between the aldehyde group in the 3'-end of the nucleotide chain and an amino group existing in fluorescein cadaverine. Thereafter, sodium borohydride was added thereto to a concentration of 1 mg/ml. Thereafter, 100 µl of the reaction solution was reacted at 4°C for a day anda night, so as to reduce the Schiff base, thereby stabilizing the bond.

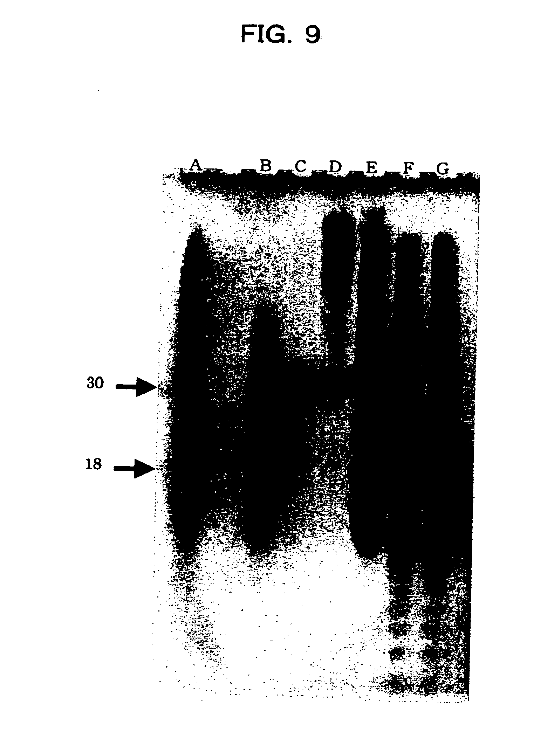

The results obtained by tracing this modification process according to 7 mol/l urea-polyacrylamide gel electrophoresis (Molecular Cloning 2nd edition, p. 11.23, 1989) are shown in FIG. 9. In the figure, the numbers and arrows indicated on the left side represent the electrophoretic positions of nucleotide chains each having the number of bases. The nucleotide chain in the gel was stained according to the silver staining method (Electrophoresis, Vol. 4, p. 92, 1983).

Lanes A, B, and C indicate a molecular weight marker, oligodeoxycytosine, and an untreated nucleotide chain, respectively. Lane D indicates a nucleotide chain, to the 3'-terminal side of which a nucleotide sequence including hypoxanthine was tailed. The number of nucleotides increased to 100 or more. Lane E indicates a nucleotide chain to which oligodeoxycytosine was annealed. Lane F indicates a nucleotide chain, at the 3'-end of which an aldehyde group was formed. The number of nucleotides decreased to 30. Lane G indicates a nucleotide chain, 3'-end of which was modified with fluorescein cadaverine and thus stabilized.

In lane D, the tailing of nucleotides having hypoxanthine is not observed, but it is seen that an unreacted nucleotide chain somewhat remains. However, such an unreacted nucleotide chain can easily be removed by extension of the tailing reaction time, an increase in the amount of terminal deoxynucleotidyltransferase added, or the like.

Since amino groups are also present in adenine, guanine, and cytosine contained in the nucleotide chain as a target for modification, there is the possibility that a reaction occurs in the nucleotide chain, thereby causing self-aggregation. However, as shown in lane F, such self-aggregation was not found. If necessary, amino groups contained in the nucleotide chain can be protected with suitable protecting groups used in the chemical synthesis of nucleotide chains, such as a benzoyl group or an isobutyryl group.

### (Example 2)

The nucleotide chain in each step that was carried out by electrophoresis in Example 1 was transferred to a nylon membrane (manufactured by Schleicher & Schuell) according to the method of Southern (J. Mol. Biol. Vol. 98, p. 503, 1975).

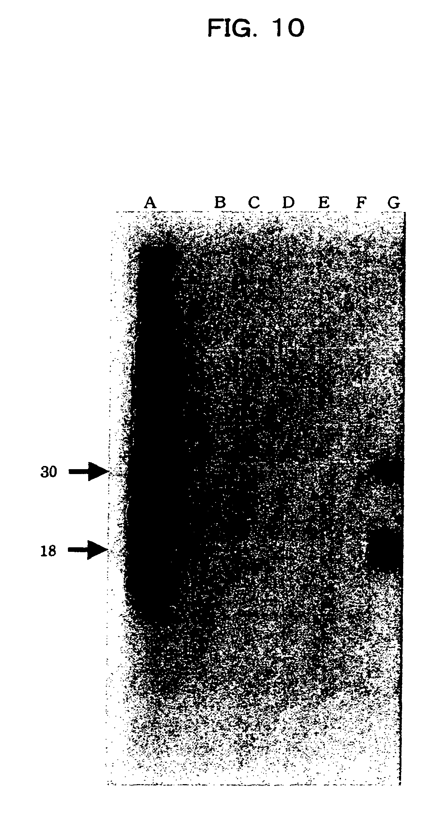

A horseradish peroxidase conjugated anti-fluorescein antibody (manufactured by Amersham Biosciences) was added dropwise to the nucleotide chain on the nylon membrane, so as to detect the presence or absence of fluorescein by exposure of a film (manufactured by Amersham Biosciences; Hyperfilm ECL) according to the chemoluminescence method (manufactured by Amersham Biosciences; ECL Detection Reagents). The results are shown in FIG. 10. In the figure, the numbers and arrows indicated on the left side represent the positions of nucleotide chains each having the number of bases, as in the case of FIG. 9.

Lanes A indicates a molecular weight marker. It is obtained by the tailing treatment with terminal deoxynucleotidyltransferase of fluorescein labeled-2',3'-dideoxyuridine-5'-triphosphate (manufactured by EnzoDiagnostics) that has previously been labeled with fluorescein.

No luminescence is observed in lanes B to F, and thus it is found that the nucleotide chain has not been modified with fluorescein.

In lane G, luminescence is detected in the nucleotide chain consisting of 30 nucleotides. Thus, it is found that the modified nucleotide chain has actually been modified with fluorescein cadaverine.

In lane G, oligodeoxycytosine consisting of 18 nucleotides is also modified with fluorescein cadaverine. This is because since oligodeoxycytosine was randomly annealed to the nucleotide chain portion of the tailed hypoxanthine, a double strand was not partially formed, and a portion on which 3-methyladenine DNA glycosylase II did not act thereby existed. Due to the presence of such a portion, oligodeoxyhypoxanthine consisting of a few nucleotides was generated, and an aldehyde group was formed at the 3'-end thereof. The aldehyde group was modified with fluorescein cadaverine. During these reactions, oligodeoxyhypoxanthine was reannealed to oligodeoxycytosine. However, such unnecessary oligodeoxycytosine can easily be separated and removed by annealing with oligodeoxyhypoxanthine or oligodeoxyguanine that has been immobilized according to gel chromatography, ion exchange chromatography, or a method suitable for fine particles such as polystyrene beads, or oligonucleotides obtained by mixing the above components.

As described in Examples 1 and 2, the 3'-terminus of a nucleotide chain can directly be modified with a modifier according to the method.

The structure and length of a nucleotide chain, a modifier, reaction conditions, a reaction composition, and the like described in each of the aforementioned examples, are all given as examples. Accordingly, such conditions are not limited to these examples, but can be modified as appropriate.

### (Example 3)

A single-stranded deoxynucleotide chain consisting of 20 nucleotides (procured from Sigma-Genosys) was used as a nucleotide chain as a target for modification. This single-stranded deoxynucleotide chain corresponds to positions 5831 to 5850 of the cDNA sequence of human skeletal muscle myosin heavy chain 1 (Genbank, the National Center of Biotechnology Information, U.S.A., Internet website:
http://www.ncbi.nlm.nih.gov/Genbank/index.html, Accession No. NM_005963 as of February 2004). A single-stranded deoxynucleotide chain consisting of 29 nucleotides (procured from Sigma-Genosys) was used as a nucleotide chain complementary to the above nucleotide chain.
Aminoxymethylcarbonylhydrazone-Cy5 was used as a modifier. These nucleotide chains have the following nucleotide sequences:
Nucleotide chain as a target for modification
TGCTG AAAGG TGACC AAAGA (Sequence 2)
Complementary nucleotide chain
ATCGGATCCTCTTTGGTCACCTTTCAGCA (Sequence 3)

### Step 1

A nucleotide chain as a target for modification (hereinafter referred to as MYH1) and a complementary nucleotide chain (hereinafter referred to as MYH1-BamHI) were suspended in a buffer solution (100 µl in total) consisting of 50 mM sodium chloride, 10 mM magnesium chloride, 1 mM dithiothreitol, and 10 mM Tris-HCl (pH 7.5), to final concentrations of 10 µM and 20 µM, respectively. The obtained mixture was heated at 65°C for 10 minutes. Thereafter, the resultant was cooled to a room temperature over 30 minutes or more, so as to form a double strand wherein the 5'-terminal region (CTTTCAGCA) of cMYH1-BamHIl protruded.

### Step 2

The formed double-stranded nucleotide was recovered by ethanol precipitation. It was then added to a solution consisting of 10 mM dITP, 10 mM dATP, 10 mM dTTP, 10 mM dCTP, 50 mM sodium chloride, 20 mM magnesium chloride, and 40 mM Tris-HCl (pH 7.5). Thereafter, modified T7 DNA polymerase Sequenase ver. 2.0 (USB) was added thereto to a final concentration of 0.5 u/µl (100 µl in total). The mixture was reacted at 37°C for 4 hours, so as to add a complementary nucleotide sequence including hypoxanthine to MYH1-BamHI in the 3'-terminus of MYH1, thereby forming a complete double-stranded nucleotide consisting of 29 base pairs. MYH1 formed in this step is indicated with (Sequence 1)
-HxHxATCCHxAT.

### Step 3

The formed double-stranded nucleotide was recovered by ethanol precipitation. It was then added to a solution consisting of 1 mM ethylenediaminetetraacetic acid, 1 mM ethylene glycol-bis(2-aminoethylether)tetraacetic acid (= glycoletherdiaminetetraacetic acid), 1 mM dithiothreitol, and 10 mM 4-(2-hydroxyethyl)piperazin-1-ethanesulfonic acid-potassium hydroxide (pH 7.4). Thereafter, 3-methyladenine DNA glycosylase II (Trevigen) was added thereto to a final concentration of 0.2 u/µl (100 µl in total). The mixture was reacted at 37°C for a day and a night, so as to hydrolyze a glycosidic bond portion of deoxyribose having hypoxanthine, thereby adding an aldehyde group to the 3'-end of MYH1. This MYH1 is indicated with (Sequence 1) -CHO.

### Step 4

A double-stranded nucleotide having an aldehyde group in the end of MYH1 was added to a solution of 50 mM 4-(2-hydroxyethyl)piperazin-1-ethanesulfonic acid-sodium hydroxide (pH 7.2). Thereafter, aminoxymethylcarbonylhydrazone-Cy5 was added as a modifier to the above solution to a final concentration of 240 µM (100 µl in total). The mixture was reacted at a room temperature for a day and a night, so as to form a Schiff base between the aldehyde group existing at the 3'-end of MYH1 and amine contained in Cy5. This MYH1 is indicated with (Sequence 1) -CHHNOCHNHNH-Cy5.

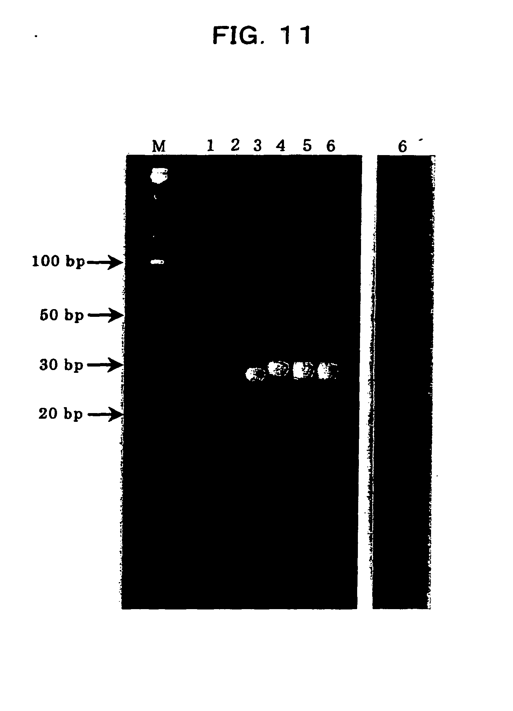

FIG. 11 shows the results of 16% polyacrylamide gel electrophoresis (with a buffer solution consisting of 90 mM Tris-boric acid and 2 mM ethylenediaminetetraacetic acid) performed on the nucleotide chain in each of the above steps. The nucleotide chain in the gel was stained with SYBR Gold (Molecular Probes). In the figure, M represents a molecular weight marker. 20 bp, 30 bp, 50 bp, and 100 bp represent the electrophoretic migrated positions of 20 base pairs, 30 base pairs, 50 base pairs, and 100 base pairs of the double-stranded nucleotide, respectively.

The band in lane 1 indicates the nucleotide chain as a target for modification (MYH1) ; the band in lane 2 indicates the complementary nucleotide chain (MYH1-BamHI); the band which appeared at the position of approximately 30 base pairs in lane 3 indicates a partial double-stranded nucleotide formed in step 1; the band in lane 4, the migration of which is slightly slower than that in lane 3, indicates a complete double-stranded nucleotide consisting of 29 base pairs formed in step 2; the band in lane 5, the mobility of which is somewhat faster than that in lane 4, indicates a double-stranded nucleotide wherein an aldehyde group has been formed on MYH1; and the band in lane 6 indicates a double-stranded nucleotide wherein MYH1 has been modified with a modifier.

There was no significant difference between lane 5 and lane 6 in terms of mobility. After completion of the electrophoresis, the double-stranded nucleotide in each lane was transferred to a nylon membrane (Schleicher & Schuell) according to the method of Southern (J. Mol. Biol., Vol. 98, p. 503, 1975). Using a horseradish peroxidase conjugated anti-cyanine antibody (manufactured by Rockland), the presence or absence of chemoluminescence was examined on a film (product name: Hyperfilm ECL, Amersham Biosciences) according to a luminol reaction. As a result, it was found that film exposure took place only with the double-stranded nucleotide in lane 6 (lane 6') . This result shows that the anti-cyanine antibody labeled with horseradish peroxidase bound to the double-stranded nucleotide in lane 6, that is, aminoxymethylcarbonylhydrazone-Cy5, to which the above antibody is able to bind, bound to MYH1.

Aminoxymethylcarbonylhydrozone-Cy5 (cyanine dye) used as a modifier was synthesized and purified according to the method of Ide et al. (Biochemistry, Vol. 32, p. 8276, 1993) and the method of Gruber et al. (Bioconjugate Chemistry, Vol. 11, p. 161, 2000), using Cy5-hydrazine (Amersham Biosciences) as a starting substance, in the following process.

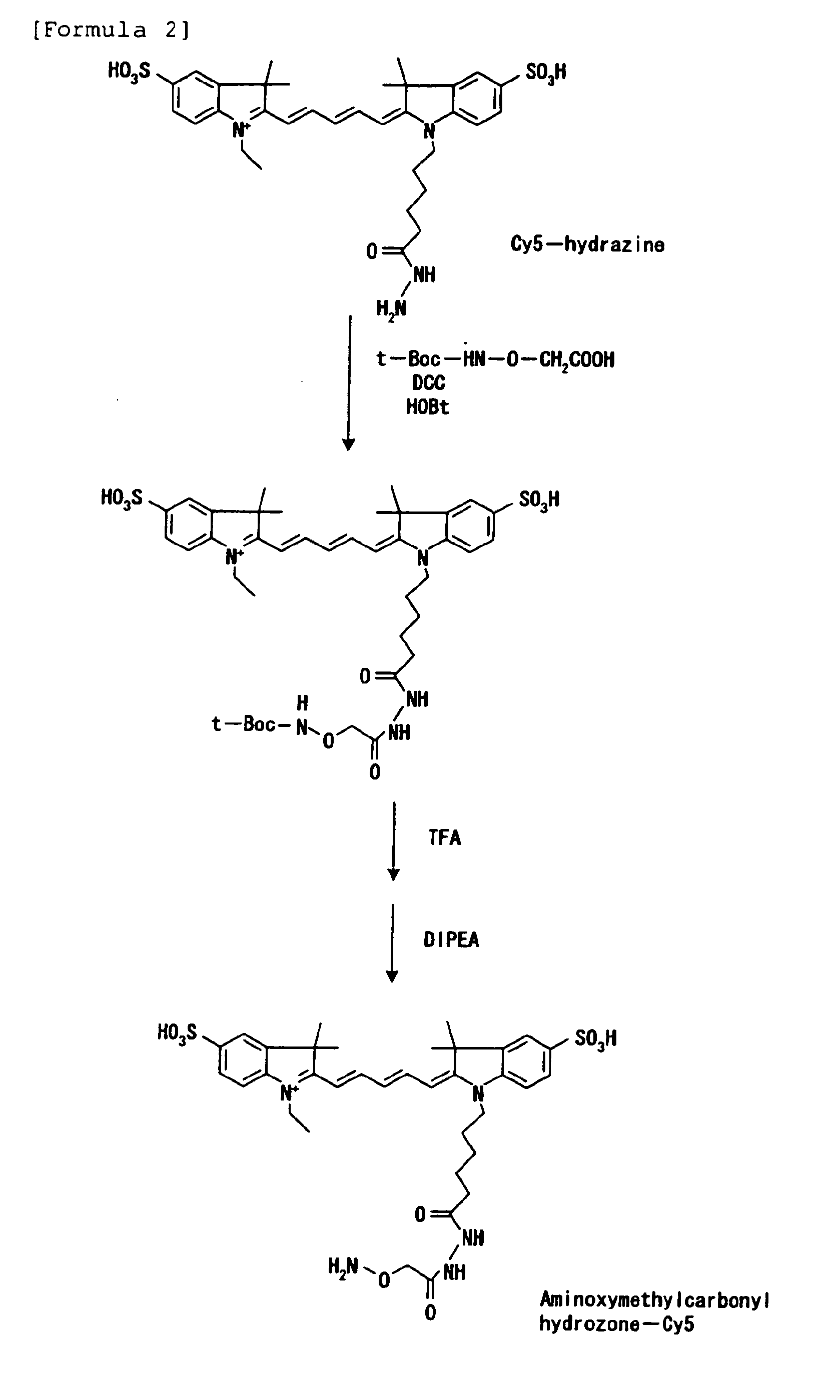

1 mM Cy5-hydrazine was allowed to react with 3 mM tert-butoxycarbonyl aminoxyacetic acid (t-Boc-NH-O-CH₂COOH) (Fluka) in the presence of 3 mM N,N'-dicyclohexylcarbodiimide (DCC) and 3 mM 1-hydroxy-1H-benzothiazole (HOBt) (300 µl in total) at 4°C for a day and a night for condensation. Thereafter, this reactionmixture was concentrated, and 50% trifluoroacetic acid (TFA) (2000 µl) was added to the obtained concentrate. The obtained mixture was reacted at a room temperature for 1 hour, so as to cleave a tert-butoxycarbonyl group (t-Boc) in the condensate. Finally, the reaction mixture was neutralized with 5% N,N'-diisopropylethylamine (DIPEA), followed by purification with a C₁₈ silica gel column, so as to obtain a compound of interest. The yield was found to be 13.4%.

As stated above, according to the present invention, the 3'-terminus of a single-stranded nucleotide, or the 3'-terminus of either one or both chains of a double-stranded nucleotide, can directly be modified with a modifier, regardless of the length of the nucleotide chain. By selecting, as a defined base, a base that does not exist in a host chain portion, for example, a base that does not originally exist as a gene and is not incorporated in a nucleotide chain during PCR amplification, the decomposition of the host chain portion can be prevented. This is a method for directly and simply modifying the 3'-terminal side of a nucleotide chain with any given modifier quantitatively and stably, regardless of the base structure of the nucleotide chain (host chain portion) or the length thereof.

Accordingly, this method enables specific labeling or conjugating of a nucleotide chain having gene information of interest. In addition, when the method aims at immobilization of a nucleotide chain, it enables stable and strong immobilization of a modifier as a linker, thereby preventing pseudo-results of gene analysis.

### Industrial Applicability

The method for modifying a nucleotide chain of the present invention enables a quantitative and stable modification of the 3'-terminus of a nucleotide chain as a target for modification with any given modifier, regardless of whether or not the nucleotide chain is a single strand or a double strand. Thus, this method is useful for gene analysis, which requires labeling, conjugating, or immobilization of a nucleotide chain.

## Claims

1. A method for modifying a nucleotide chain, the method comprising:
allowing a nucleotide chain as a target for modification to be degraded by a catabolic enzyme that reacts specifically with a defined base composition, said nucleotide chain including in a 3'-terminal region thereof a nucleotide sequence containing the defined base composition; and
forming a functional group in said nucleotide chain at a 3'-terminal thereof, the functional group being capable of binding a required modifier to the 3'-terminal of said nucleotide chain as a target for modification, wherein hypoxanthine is used as a defined base composition, wherein 3-methyladenine DNA glycosylase is used as a catabolic enzyme and wherein the functional group is an aldehyde group.

2. The method for modifying a nucleotide chain according to claim 1, wherein the defined base composition is located in the 3'-terminal region of a nucleotide chain to be modified, but said defined base composition is not found in the principal sequence in said nucleotide chain.

3. The method for modifying a nucleotide chain according to claim 1 or 2, wherein a nucleotide sequence containing the defined base composition is added to the 3'-terminal region of said nucleotide chain as a target for modification.

4. The method for modifying a nucleotide chain according to claim 3, wherein a nucleotide sequence containing the defined base composition is added to the 3'-terminal region of the nucleotide chain as a target for modification with a tailing method.

5. The method for modifying a nucleotide chain according to claim 3, wherein a nucleotide chain as a target for modification is annealed with a single stranded nucleotide chain having a complementary sequence in the 3'-terminal region thereof, so that the defined base composition is added to said nucleotide chain as a target for modification in the 3'-terminal region.

6. The method for modifying a nucleotide chain according to claim 3, wherein at least either one single stranded nucleotide chain, as a target for modification, of a double stranded chain is cleaved with a restriction enzyme that specifically recognizes a nucleotide sequence in the 3'-terminal region and a complementary sequence containing the defined base composition is added to said cleaved nucleotide chain as a target for modification in the 3'-terminal region.

7. The method for modifying a nucleotide chain according to claim 3, wherein at least either one single stranded nucleotide chain, as a target for modification, of a double stranded chain is ligated to another double stranded nucleotide chain containing a defined base composition in a 5'-terminal of the nucleotide sequence using DNA ligase, so that the nucleotide sequence containing a defined base is added to said nucleotide chain as a target for modification in the 3'-terminal.

8. The method for modifying a nucleotide chain according to claim 5 or 6, wherein addition of a nucleotide sequence is carried out by incorporation of nucleotides into a nucleotide chain as a target for modification.

9. The method for modifying a nucleotide chain according to claim 1, wherein a nucleotide chain containing the defined base composition in the sequence is synthesized chemically.

10. The method for modifying a nucleotide chain according to claim 1, wherein a complementary nucleotide sequence against the defined base composition is added prior to a catabolic enzyme treatment.

11. The method for modifying a nucleotide chain according to claim 1, wherein the modifier has an amino group that is capable of binding to a functional group formed in the 3'-terminal of a nucleotide chain.

12. The method for modifying a nucleotide chain according to claim 1 or 11, wherein the modifier is a material for labeling or conjugating a nucleotide chain.

13. The method for modifying a nucleotide chain according to claim 12, wherein the modifiers are fluorescence substances, vitamins, lipids, amino acids, oligopeptides, proteins, or exogenous substances.

14. The method for modifying a nucleotide chain according to claim 1 or 11, wherein the modifier is a material capable of binding to a substrate used for gene analysis.

15. The method for modifying a nucleotide chain according to claim 14, wherein the modifiers are amino-alkanethiols or amino-silane coupling reagents.

## Patentansprüche

1. Verfahren zum Modifizieren einer Nukleotidkette, wobei das Verfahren umfasst:
das Gestatten eines Abbaus einer Nukleotidkette als ein Ziel für eine Modifizierung durch ein katabolisches Enzym, welches spezifisch mit einer definierten Basenzusammensetzung reagiert, wobei die Nukleotidkette in einer 3'-terminalen Region davon eine Nukleotidsequenz einschließt, welche die definierte Basenzusammensetzung enthält; und
das Bilden einer funktionellen Gruppe in der Nukleotidkette an einem 3'-Ende davon, wobei die funktionelle Gruppe ein erforderliches Modifizierungsagenz an das 3'-Ende der Nukleotidkette als ein Ziel für eine Modifizierung binden kann, wobei Hypoxanthin als eine definierte Basenzusammensetzung verwendet wird, wobei 3-Methyladenin-DNA-Glycosylase als ein katabolisches Enzym verwendet wird und wobei die funktionelle Gruppe eine Aldehydgruppe ist.

2. Verfahren zum Modifizieren einer Nukleotidkette nach Anspruch 1, worin die definierte Basenzusammensetzung in der 3'-terminalen Region einer zu modifizierenden Nukleotidkette liegt, aber die definierte Basenzusammensetzung nicht in der Hauptsequenz in der Nukleotidkette vorkommt.

3. Verfahren zum Modifizieren einer Nukleotidkette nach Anspruch 1 oder 2, worin eine Nukleotidsequenz, welche die definierte Basenzusammensetzung enthält, an die 3'-terminale Region der Nukleotidkette als ein Ziel für eine Modifizierung angefügt wird.

4. Verfahren zum Modifizieren einer Nukleotidkette nach Anspruch 3, worin eine Nukleotidsequenz, welche die definierte Basenzusammensetzung enthält, an die 3'-terminale Region der Nukleotidkette als ein Ziel für eine Modifizierung mit einem Tailing-Verfahren angefügt wird.

5. Verfahren zum Modifizieren einer Nukleotidkette nach Anspruch 3, worin eine Nukleotidkette als ein Ziel für eine Modifizierung mit einer einzelsträngigen Nukleotidkette mit einer komplementären Sequenz in der 3'-terminalen Region davon hybridisiert wird, so dass die definierte Basenzusammensetzung an die Nukleotidkette als ein Ziel für eine Modifizierung in der 3'-terminalen Region angefügt wird.

6. Verfahren zum Modifizieren einer Nukleotidkette nach Anspruch 3, worin wenigstens eine einzelsträngige Nukleotidkette, als ein Ziel für eine Modifizierung, von einer doppelsträngigen Kette mit einem Restriktionsenzym gespalten wird, welches eine Nukleotidsequenz in der 3'-terminalen Region spezifisch erkennt, und eine komplementäre Sequenz, welche die definierte Basenzusammensetzung enthält, an die gespaltene Nukleotidkette als ein Ziel für eine Modifizierung in der 3'-terminalen Region angefügt wird.

7. Verfahren zum Modifizieren einer Nukleotidkette nach Anspruch 3, worin wenigstens eine einzelsträngige Nukleotidkette, als ein Ziel für eine Modifizierung, von einer doppelsträngigen Kette an eine andere doppelsträngige Nukleotidkette, die eine definierte Basenzusammensetzung in einem 5'-Ende der Nukleotidsequenz enthält, unter Verwendung von DNA-Ligase ligiert wird, so dass die Nukleotidsequenz, die eine definierte Base enthält, an die Nukleotidkette als ein Ziel für eine Modifizierung in dem 3'-Ende angefügt wird.

8. Verfahren zum Modifizieren einer Nukleotidkette nach Anspruch 5 oder 6, worin das Anfügen einer Nukleotidsequenz durch Einbau von Nukleotiden in eine Nukleotidkette als ein Ziel für eine Modifizierung erfolgt.

9. Verfahren zum Modifizieren einer Nukleotidkette nach Anspruch 1, worin eine Nukleotidkette, welche die definierte Basenzusammensetzung in der Sequenz enthält, chemisch synthetisiert wird.

10. Verfahren zum Modifizieren einer Nukleotidkette nach Anspruch 1, worin eine komplementäre Nukleotidsequenz gegen die definierte Basenzusammensetzung vor einer Behandlung mit einem katabolischen Enzym angefügt wird.

11. Verfahren zum Modifizieren einer Nukleotidkette nach Anspruch 1, worin das Modifizierungsagenz eine Aminogruppe aufweist, welche an eine funktionelle Gruppe binden kann, die in dem 3'-Ende einer Nukleotidkette gebildet ist.

12. Verfahren zum Modifizieren einer Nukleotidkette nach Anspruch 1 oder 11, worin das Modifizierungsagenz ein Material zum Markieren oder Konjugieren einer Nukleotidkette ist.

13. Verfahren zum Modifizieren einer Nukleotidkette nach Anspruch 12, worin die Modifizierungsagenzien Fluoreszenzsubstanzen, Vitamine, Lipide, Aminosäuren, Oligopeptide, Proteine oder exogene Substanzen sind.

14. Verfahren zum Modifizieren einer Nukleotidkette nach Anspruch 1 oder 11, worin das Modifizierungsagenz ein Material ist, das an ein für eine Genanalyse verwendetes Substrat binden kann.

15. Verfahren zum Modifizieren einer Nukleotidkette nach Anspruch 14, worin die Modifizierungsagenzien Aminoalkanthiole oder Aminosilan-Kupplungsreagenzien sind.

## Revendications

1. Procédé de modification d'une chaîne nucléotidique, le procédé comprenant :
la dégradation d'une chaîne nucléotidique comme une cible pour une modification par une enzyme catabolique qui réagit spécifiquement avec une composition de base définie, ladite chaîne nucléotidique incluant dans une région d'extrémité 3' de celle-ci une séquence nucléotidique contenant la composition de base définie ; et
la formation d'un groupe fonctionnel dans ladite chaîne nucléotidique à une extrémité 3' de celle-ci, le groupe fonctionnel étant apte à lier un modificateur requis à l'extrémité 3' de ladite chaîne nucléotidique comme une cible pour une modification, dans lequel l'hypoxanthine est utilisée comme une composition de base définie, dans lequel la 3-méthyladénine ADN glycosylase est utilisée comme une enzyme catabolique et dans lequel le groupe fonctionnel est un groupe aldéhyde.

2. Procédé de modification d'une chaîne nucléotidique selon la revendication 1, dans lequel la composition de base définie est située dans la région d'extrémité 3' d'une chaîne nucléotidique destinée à être modifiée, mais ladite composition de base définie ne se trouve pas dans la séquence principale dans ladite chaîne nucléotidique.

3. Procédé de modification d'une chaîne nucléotidique selon la revendication 1 ou 2, dans lequel une séquence nucléotidique contenant la composition de base définie est ajoutée à la région d'extrémité 3' de ladite chaîne nucléotidique comme une cible pour une modification.

4. Procédé de modification d'une chaîne nucléotidique selon la revendication 3, dans lequel une séquence nucléotidique contenant la composition de base définie est ajoutée à la région d'extrémité 3' de la chaîne nucléotidique comme une cible pour une modification avec un procédé d'extension.

5. Procédé de modification d'une chaîne nucléotidique selon la revendication 3, dans lequel une chaîne nucléotidique comme une cible pour une modification est annelée avec une chaîne nucléotidique à brin unique ayant une séquence complémentaire dans la région d'extrémité 3' de celle-ci, de sorte que la composition de base définie est ajoutée à ladite chaîne nucléotidique comme une cible pour une modification dans la région d'extrémité 3'.

6. Procédé de modification d'une chaîne nucléotidique selon la revendication 3, dans lequel au moins l'une ou l'autre chaîne nucléotidique à brin unique, comme une cible pour une modification, d'une chaîne à double brin est clivée avec une enzyme de restriction qui reconnaît spécifiquement une séquence nucléotidique dans la région d'extrémité 3' et une séquence complémentaire contenant la composition de base définie est ajoutée à ladite chaîne nucléotidique clivée comme une cible pour une modification dans la région d'extrémité 3'.

7. Procédé de modification d'une chaîne nucléotidique selon la revendication 3, dans lequel au moins l'une ou l'autre chaîne nucléotidique à brin unique, comme une cible pour une modification, d'une chaîne à double brin est ligaturée à une autre chaîne nucléotidique à double brin contenant une composition de base définie dans une extrémité 5' de la séquence nucléotidique en utilisant une ADN ligase, de sorte que la séquence nucléotidique contenant une base définie est ajoutée à ladite chaîne nucléotidique comme une cible pour une modification dans l'extrémité 3'.

8. Procédé de modification d'une chaîne nucléotidique selon la revendication 5 ou 6, dans lequel l'addition d'une séquence nucléotidique est exécutée par incorporation de nucléotides dans une chaîne nucléotidique comme une cible pour une modification.

9. Procédé de modification d'une chaîne nucléotidique selon la revendication 1, dans lequel une chaîne nucléotidique contenant la composition de base définie dans la séquence est synthétisée chimiquement.

10. Procédé de modification d'une chaîne nucléotidique selon la revendication 1, dans lequel une séquence nucléotidique complémentaire contre la composition de base définie est ajoutée avant un traitement par enzyme catabolique.

11. Procédé de modification d'une chaîne nucléotidique selon la revendication 1, dans lequel le modificateur comporte un groupe amino qui est apte à se lier à un groupe fonctionnel formé dans l'extrémité 3' d'une chaîne nucléotidique.

12. Procédé de modification d'une chaîne nucléotidique selon la revendication 1 ou 11, dans lequel le modificateur est une matière pour marquer ou conjuguer une chaîne nucléotidique.

13. Procédé de modification d'une chaîne nucléotidique selon la revendication 12, dans lequel les modificateurs sont des substances à fluorescence, des vitamines, des lipides, des acides aminés, des oligopeptides, des protéines, ou des substances exogènes.

14. Procédé de modification d'une chaîne nucléotidique selon la revendication 1 ou 11, dans lequel le modificateur est une matière apte à se lier à un substrat utilisé pour une analyse génique.

15. Procédé de modification d'une chaîne nucléotidique selon la revendication 14, dans lequel les modificateurs sont des amino-alcanethiols ou des réactifs de couplage amino-silane.
